# EUROPEAN PATENT APPLICATION

(11) **EP 0 627 234 A1**
(43) Date of publication of application: **07.12.1994**
(21) Application number: 94105719.2
(22) Date of filing: 13.04.1994
(51) Int. Cl.: A61N 1/05, A61N 1/39

(54) **Implantable electrode system**

(30) Priority: 01.06.1993 SE 9301857
(71) Applicant: Pacesetter AB, S-171 95 Solna (SE)
(72) Inventor: Strandberg, Hans, S-172 36 Sundbyberg (SE); Hirschberg, Jakub, S-183 44 Täby (SE)
(74) Representative: Lettström, Richard Wilhelm

(57) **Abstract**

The invention relates to an implantable electrode system (3) intended to be connected at a proximal end (4) to a medical apparatus (1) for stimulating living tissue (2), comprising a flexible, insulating sleeve, a first conductor (5) running in the sleeve from the proximal end to the distal end, a tip electrode (8) located at the distal end (7) of the sleeve and connected to the first conductor and an indifferent electrode with a large electrode surface relative to the tip electrode. In order to achieve the largest possible surface for the indifferent electrode, the indifferent electrode (9) is devised as part of the insulating sleeve on a defined section of the insulating sleeve's length between the distal end (7) and the proximal end (4). The electrode system's (2) flexibility is accordingly retained while an indifferent electrode (9) of optional size is achieved.

## Description

The invention relates to an implantable electrode system intended to be connected at a proximal end to a medical apparatus for stimulating living tissue, comprising a flexible, insulating sleeve, a first conductor running in the sleeve from the proximal end to a distal end, a tip electrode located at the distal end of the sleeve and connected to the first conductor, an indifferent electrode with a large electrode surface relative to the tip electrode and a second conductor, electrically insulated from the first conductor, connected to the indifferent electrode and to the medical apparatus, whereby electrical stimulation pulses can be emitted by the medical apparatus to the living tissue across the tip electrode and the indifferent electrode, and electrical signals in the living tissue can be sensed between the tip electrode and the indifferent electrode by the medical apparatus.

In US-A-4-4,387,717 is described a cardiological electrode system for stimulating a heart and sensing its activity. The system comprises a first conductor which runs through an insulating sleeve to a tip electrode placed in the heart. A ring electrode is located at a defined distance from the tip electrode. The ring electrode is connected to a second conductor. The first conductor and the second conductor are connected, in turn, to a pacemaker. The ring electrode serves as an indifferent electrode and can be placed at a distance from the tip electrode so the former is positioned inside or outside the heart.

Especially in the sensing of cardiac activity, it is important that no signals be detected by the indifferent electrode, since these signals could interfere with the interpretation of signals detected by the tip electrode. The indifferent electrode should also have a large surface in order to reduce the density of current near it, so as to prevent needless stimulation of body tissue around the indifferent electrode when the medical apparatus emits a stimulation pulse across the tip electrode and the indifferent electrode. The described ring electrode cannot be devised with a large electrode surface, since it is stiff and can therefore not be allowed to have a large size, as this would make the entire electrode system stiff and impossible to implant.

A large surface for the indifferent electrode can be achieved when e.g. the pacemaker enclosure is devised as the indifferent electrode. This has been made, according to the aforementioned document, on prior art pacemakers. As a result, however, the indifferent electrode can detect muscle signals from nearby muscle tissue. This noise would then interfere with the interpretation of heart signals.

Alternately, a separate, large-surface electrode could be applied outside the heart, apart from the electrode system inside the heart, as is also described in the aforementioned document. However, this kind of indifferent electrode leads to complications in implantation and can also be difficult to affix at a suitable site without muscle signals being detected by it.

A ring electrode according to the known electrode system has, as noted above, a small surface, since the lead must be flexible and compliant when the system is implanted and to prevent damage to the vascular system after implantation.

In US-A-3,915,174 is described an electrode system which, in addition to a tip electrode and a ring electrode, is also provided with a large-surface indifferent electrode along the length of an electrode catheter. The indifferent electrode is placed on the electrode catheter in such a way that it is outside the heart after the electrode system has been implanted. The document relates that the indifferent electrode can be allowed to extend down into the heart when its size, in principle, encompasses the entire electrode catheter. The indifferent electrode consists of a cylindrical ring made of a conductive material.

One object of the invention is to achieve an implantable electrode system which solves the above-described problems, which is easy to manufacture and implant and which minimizes the risk of damage to the vascular system after implantation.

One such electrode system is achieved in accordance with the present invention in that the sleeve is devised to be conductive on a defined section of its length between the distal end and the proximal end in order to serve as the indifferent electrode.

Utilization of an optional section of the sleeve for the indifferent electrode and devising that section to be electrically conductive results in an electrode system which solves the problems. The indifferent electrode acquires a large electrode surface with a position which minimizes the risk of any interference signals being detected. Since the sleeve itself is made conductive, fabrication of the electrode system is facilitated. The indifferent electrode is flexible, so implantation of the electrode system is not impeded, and the vascular system is subjected to minimum trauma after implantation. In addition, the diameter of the electrode system between the indifferent electrode and the sleeve does not change, and sharp edges which could damage tissue are completely avoided.

In this context, it is advantageous if the distal end is devised to be introducible into a heart and the indifferent electrode had a size enabling at least a part of the indifferent electrode to be outside the heart after the distal end has been introduced into the heart.

Since part of the indifferent electrode is outside the heart and the indifferent electrode has an area which is larger than the area of the above-described ring electrode, the indifferent electrode according to the invention does not detect any signals from the heart. Nor does it detect any muscle signals, since it is located in the vascular system. In this manner, signals, detected by the tip electrode can be easily interpreted.

One refinement of the electrode system is achieved in accordance with the invention in that the indifferent electrode comprises a conductive coating on the defined section of the sleeve, and the conductive coating is connected to the second conductor.

This makes the sleeve insulating along its entire interior length and conductive on its exterior in the given section. This therefore reduces the risk of any short-circuit developing between the first conductor and the indifferent electrode.

It is advantageous if the electrode system is devised so the indifferent electrode is made of a conductive polymer. The conductive polymer will have the same flexible properties as the sleeve and will therefore not restrict the electrode system's flexibility and compliance.

An alternative embodiment is achieved in accordance with the invention in that the sleeve is doped with a conductive material on the defined section of sleeve.

When the sleeve is doped with a conductive material, preferably a biocompatible metal or metallic compound such as titanium nitride or platinum, or some other biocompatible conductor such as carbon, the flexible properties of the electrode system are retained while electrical conductivity is achieved for the sleeve.

Another embodiment of the system is achieved in accordance with the invention in that a second insulating sleeve is placed inside the first insulating sleeve, the second conductor is helically coiled in the space between the sleeves and the first conductor is helically coiled inside the second sleeve.

The first conductor, which leads to the tip electrode, is hereby well-protected and well-insulated from the second conductor and ambient body fluids.

The invention will now be described below in greater detail, referring to six figures in which
- FIG. 1: shows an electrode system, according to the invention, connected to a pacemaker and a heart respectively;
- FIG. 2: shows a first embodiment of a way the indifferent electrode can be devised; and
- FIG. 3: shows a second embodiment of a way the indifferent electrode can be devised.

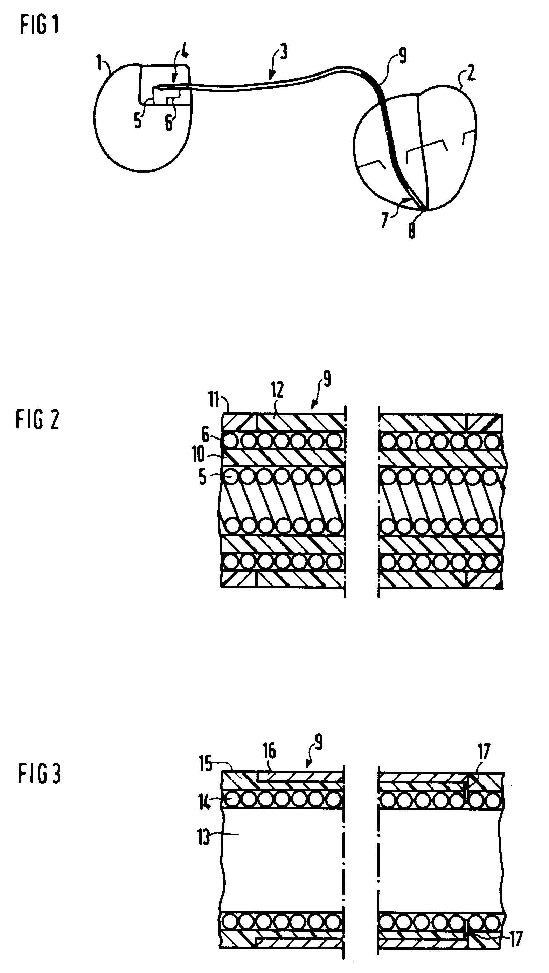

In FIG. 1 is shown a pacemaker 1 connected to a heart 2 via an electrode system 3. The electrode system 3 is connected to the pacemaker 1 at a proximal end 4, a first conductor 5 and a second conductor 6 being connected to electronics in the pacemaker 1. The electrode system 3 is further connected to the heart at a distal end 7. A tip electrode 8, which is connected to the first conductor 5 and to heart tissue, is on the distal end 7. An indifferent electrode 9, insulated from the tip electrode 8 and designated in the figure by the filled-in part of the electrode system 3, is provided. The indifferent electrode 9 is connected to the second conductor 6. The pacemaker 1 can, via the first conductor 5 and the second conductor 6, emit and receive electrical signals to/from the heart 2. When electrical signals are sensed in the heart 2, the tip electrode 8 detects the signals, and the indifferent electrode 9 remains passive, so signals obtained are easily interpreted by pacemaker 1 electronics. In principle, the indifferent electrode 9 can extend along any desired length of the electrode system 3. The salient point is that at least part of the indifferent electrode 9 is located outside the heart 2 so as to prevent heart signals from being detected by the indifferent electrode 9, since this would interfere with interpretation of the signals received.

In FIG. 2 is shown a first embodiment of a way of achieving the indifferent electrode 9. Only part of the electrode system 3 is shown. The first conductor 5 is helically coiled inside an insulating sleeve 10. The second conductor 6 is helically coiled around the insulating sleeve 10. The second conductor 6 is, in turn, enclosed in a second insulating sleeve 11. In order to achieve a flexible indifferent electrode 9 with a large indifferent electrode surface, part of the second sleeve 11 is made of a conductive polymer 12. The conductive polymer 12 is in electrical contact with the second conductor 6 along its entire length.

In FIG. 3 is shown a second embodiment of a way of achieving the indifferent electrode 9. As in FIG. 2, a first conductor is enclosed in an insulating sleeve 13. A second helically coiled conductor 14 is coiled around this sleeve 13. A second insulating sleeve 15 encloses the second conductor 14. In this embodiment, the second insulating sleeve 15 has been doped with a conductive, biocompatible material 16 on a defined section of the second insulating sleeve 15 so as to form the indifferent electrode 9. A connecting part 17 electrically interconnects the doped coating 16 and the second conductor 14.

Appropriate materials are e.g. biocompatible metals and metallic compounds, such as platinum and titanium nitride, or some other biocompatible conductive material, such as carbon. Since the conductive coating 16 consists of doped section of sleeve 15 and is not made of solid metal, it is sufficiently flexible for use, in principle, along the entire length of the electrode system 3.

The described embodiments can also be combined in different ways. For example, the conductive polymer 12 in FIG. 2 can be replaced with a doped section through the entire sleeve 11, and the doped section 16 in FIG. 3 can be made of a conductive polymer. The conductive polymer can, in turn, be doped so as to further improve conductivity.

## Claims

1. An implantable electrode system (3) intended to be connected at a proximal end (4) to a medical apparatus (1) for stimulating living tissue (2), comprising a flexible, insulating sleeve (11; 15), a first conductor (5) running in the sleeve (11; 15) from the proximal end (4) to the distal end (7), a tip electrode (8) located at the distal end (7) of the sleeve (11; 15) and connected to the first conductor (5), an indifferent electrode (9) with a large electrode surface relative to the tip electrode (8) and a second conductor (6; 14), electrically insulated from the first conductor (5), connected to the indifferent electrode (9) and to the medical apparatus (1), whereby electrical stimulation pulses can be emitted by the medical apparatus (1) to the living tissue (2) across the tip electrode (8) and the indifferent electrode (9), and electrical signals in the living tissue can be sensed between the tip electrode (8) and the indifferent electrode (9) by the medical apparatus (1), **characterized in that** the sleeve (11; 15) is devised to be conductive on a defined section of its length between the distal end (7) and the proximal end (4) in order to serve as the indifferent electrode (9).

2. An electrode system according to claim 1, **characterized in that** the distal end (7) is devised to be introducible into a heart (2) and the indifferent electrode (9) has a size enabling at least a part of the indifferent electrode (9) to be outside the heart (2) after the distal end (7) has been introduced into the heart.

3. An electrode system according to claim 1 or 2, **characterized in that** the indifferent electrode (9) comprises a conductive coating (16) on the defined section of the sleeve (15), and the conductive coating (16) is connected to the second conductor (14).

4. An electrode system according to any of the above claims, **characterized in that** the indifferent electrode (9) is made of a conductive polymer.

5. An electrode system according to any of the above claims, **characterized in that** the sleeve (11; 15) is doped with a conductive material on the defined section of the sleeve.

6. An electrode system according to claim 5, **characterized in that** the sleeve (11; 15) is doped with a biocompatible metal, metallic compound or carbon.

7. An electrode system according to any of the above claims, **characterized in that** an additional insulating sleeve (10; 13) is placed inside the sleeve (11; 15), the second sleeve (6; 14) is helically coiled in the space between the sleeves (10, 11; 13, 15) and the first conductor (5) is helically coiled inside the additional sleeve (10; 13).
